# EUROPEAN PATENT APPLICATION

(11) **EP 2 181 983 A1**
(43) Date of publication of application: **05.05.2010**
(21) Application number: 08791533.6
(22) Date of filing: 24.07.2008
(51) Int. Cl.: C07C 227/40, C07C 229/28, C07C 319/28, C07C 323/58, C07K 1/08

(54) **METHOD FOR SELECTIVE REMOVAL OF DIBENZOFULVENE DERIVATIVE**

(30) Priority: 25.07.2007 JP 2007193153
(71) Applicant: Ajinomoto Co., Inc., Tokyo 104-8315 (JP)
(72) Inventor: TAKAHASHI, Daisuke, Yokkaichi-shi Mie 510-0885 (JP)
(74) Representative: Nicholls, Kathryn Margaret
(86) International application number: PCT/JP2008/063281
(87) International publication number: WO 2009/014177

(57) **Abstract**

A reaction mixture containing dibenzofulvene and/or a dibenzofulvene amine adduct, which is obtained by reacting an amino acid compound protected with an Fmoc group with an amine for deprotection, is stirred and partitioned in a hydrocarbon solvent having a carbon number of 5 or above and a polar organic solvent (excluding organic amide solvents) immiscible with the hydrocarbon solvent, and the hydrocarbon solvent layer is removed, whereby dibenzofulvene and/or the dibenzofulvene amine adduct generated as a by-product after the deprotection can be efficiently removed.

## Description

### Technical Field

The present invention relates to a method of efficiently removing a dibenzofulvene derivative generated as a by-product during deprotection of an amino acid compound protected with an Fmoc (9-fluorenylmethoxycarbonyl) group and the like.

### Background Art

The Fmoc group is an amino-protecting group and is widely used since it provides many advantages such as suitable liposolubility imparted to the protected compound to facilitate handling, easy tracking of reaction utilizing imparted UV absorption, stability in the neutral to acidic range, easy deprotection under mild reaction conditions with amine and the like. Particularly, it is an important protecting group of amino group of amino acid and peptide in peptide synthesis.

During removal of the Fmoc group, dibenzofulvene and/or an adduct of dibenzofulvene with amine, which is a deprotecting reagent, (hereinafter to be sometimes referred to collectively as "dibenzofulvene derivative" in the present specification) are/is by-produced. Particularly in the peptide synthesis, the dibenzofulvene derivative needs to be efficiently removed, since it possibly causes side reactions such as 9-fluorenylmethylation and the like, when the dibenzofulvene derivative remains in the next step. However, the dibenzofulvene derivative is highly liposoluble, and cannot be removed by washing with water and the like of the reaction mixture.

In peptide synthesis, the Fmoc group is mainly utilized as a protecting group for a solid phase synthesis method, which can easily remove a dibenzofulvene derivative by washing a solid support. However, the solid phase method has problems in scaling up and reactivity, since the reaction is limited to the surface of a solid support.

On the other hand, a Boc (tert-butoxycarbonyl) group capable of removing a by-product of deprotection as a gas (isobutene, carbon dioxide) is mainly utilized for a liquid phase synthesis method of peptide, since dibenzofulvene derivative cannot be easily removed. However, when a peptide containing a sulfur-containing amino acid such as cysteine, methionine and the like is to be synthesized, what is called a Boc method, wherein a Boc group is used at the N-terminal and a Bzl group is used in combination for the protection of a functional group of an amino acid side chain and the C-terminal, cannot be employed, since the sulfur-containing amino acid poisons catalysts and the Bzl group cannot be deprotected with a catalytic reduction. Thus, use of the Fmoc group as an N-terminal protecting group is sometimes desired.
In view of such background, development of a liquid phase method permitting easy scaling up and suitable for industrial production of peptide pharmaceutical products and the like is desired, which can efficiently remove dibenzofulvene derivatives when the Fmoc group is used as a protecting group.

Non-patent document 1 describes a method for removing a dibenzofulvene derivative in the liquid phase synthesis of peptide, which includes adding a hydrocarbon solvent such as hexane and the like for trituration of a residue obtained by concentrating a reaction extract to dryness, thereby dissolving a dibenzofulvene derivative in the solvent, and isolating the deprotected peptide as crystals.
However, this method is poor in operability, sometimes fails to reproduce at a large scale, and is unsuitable for industrial production. In addition, when a desired deprotected peptide is an oily substance, this method cannot be used, since it requires crystallization of the peptide. Furthermore, the method is associated with problems of low recovery rate and the like due to dissolution of peptide itself in a hydrocarbon solvent when the peptide chain is short.

patent document 1: WO 03/018188
non-patent document 1: Jikken Kagaku Koza fifth edition, (Japan), MARUZEN CO., LTD., published on March 31, 2005, vol. 16, page 272

### Disclosure of the Invention

### Problems to be Solved by the Invention

An object of the present invention is to provide a method of efficiently removing a dibenzofulvene derivative generated as a by-product after deprotection when the Fmoc group is used as a protecting group, particularly, a method applicable to industrial production by liquid phase synthesis of peptide, and further, an operation leading to a method enabling one-pot synthesis.

### Means of Solving the Problems

The present inventors have conducted intensive studies of a method of removing a dibenzofulvene derivative in an attempt to solve the above-mentioned problems, and found that, in partitioning between a hydrocarbon solvent having a carbon number of 5 or above and a polar organic solvent immiscible with the hydrocarbon solvent, a dibenzofulvene derivative is efficiently extracted into the hydrocarbon solvent layer, and a deprotected amino acid compound is efficiently transferred to the polar solvent layer and scarcely extracted into the hydrocarbon solvent, that is, the dibenzofulvene derivative can be conveniently removed by removing the hydrocarbon solvent layer from the organic solvent-organic solvent partitioned layers, which resulted in the completion of the present invention.
Accordingly, the present invention encompasses the following.
[1] A method of removing dibenzofulvene and/or a dibenzofulvene amine adduct from a reaction mixture obtained by reacting an amino acid compound protected with an Fmoc group with an amine for deprotection, which comprises stirring and partitioning the reaction mixture in a hydrocarbon solvent having a carbon number of 5 or above and a polar organic solvent (excluding organic amide solvents) immiscible with the hydrocarbon solvent, and removing the hydrocarbon solvent layer in which the dibenzofulvene and/or the dibenzofulvene amine adduct are/is dissolved.
[2] The method of the above-mentioned [1], wherein the amine is selected from diethylamine, dimethylamine, piperidine, morpholine and 1,8-diazabicyclo[5.4.0]undecen-7-en (DBU).
[3] The method of the above-mentioned [2], wherein the amine is selected from diethylamine and dimethylamine.
[4] The method of any of the above-mentioned [1] to [3], wherein the hydrocarbon solvent is at least one kind selected from n-pentane, n-hexane, n-heptane, n-octane and decalin.
[5] The method of any of the above-mentioned [1] to [4], wherein the polar organic solvent comprises at least one kind selected from acetonitrile and methanol.
[6] The method of any of the above-mentioned [1] to [5], wherein the polar organic solvent further comprises water.
[7] The method of any of the above-mentioned [1] to [6], wherein the amino acid compound protected with the Fmoc group is an amino acid ester protected with an Fmoc group or a peptide protected with an Fmoc group.
[8] A method of producing peptide by a liquid phase synthesis method, comprising the method of the above-mentioned [7].
[9] The method of the above-mentioned [8], comprising
   (1) a step of condensing C-protected peptide or C-protected amino acid with N-Fmoc amino acid in the presence of a condensing agent, and/or
   (2) a step of condensing C-protected peptide or C-protected amino acid with N-Fmoc amino acid activated ester.
[10] The method of the above-mentioned [9], wherein the step (1) is performed in the further presence of an activator.
[11] The method of the above-mentioned [9] or [10], wherein an intermediate peptide obtained without isolation as a solid in the above-mentioned [1] is subjected to the steps of the above-mentioned [9].
[12] The method of any of the above-mentioned [8] to [11], which is performed by one-pot synthesis.

### Effect of the Invention

The present invention provides a method of conveniently removing a dibenzofulvene derivative generated as a by-product during deprotection of an Fmoc group. The method does not require complicated operations such as trituration, filtration, washing and the like of crystals, and can also be applied easily to large-scale reactions. As a result, the Fmoc group can be easily utilized for industrial production, which strikingly broadens the options of the production methods of compounds requiring protection of an amino group.
Particularly, when the method of the present invention is applied to a liquid phase synthesis method of peptide, dibenzofulvene derivatives are conveniently removed by washing alone, and peptide with an unprotected N-terminal obtained by deprotection can be conveniently purified as necessary by further combining washing operations such as washing with water, washing with basic aqueous solution, washing with acidic aqueous solution and the like. As such, a peptide elongation reaction in the next step can be continuously performed, thus enabling one-pot synthesis of peptide, which is particularly preferable for industrial production.

### Best Mode for Carrying out the Invention

The symbols and abbreviations used in the present specification mean the following.
(1) Boc: tert-butoxycarbonyl
(2) Z: benzyloxycarbonyl
(3) Fmoc: 9-fluorenylmethoxycarbonyl
(4) Bsmoc: 1,1-dioxobenzo[b]thiophen-2-ylmethoxycarbonyl
(5) Alloc: allyloxycarbonyl
(6) Ac: acetyl
(7) Me: methyl
(8) Et: ethyl
(9) iPr: isopropyl
(10) tBu: tert-butyl
(11) Bzl: benzyl
(12) Fm: 9-fluorenylmethyl
(13) Trt: trityl
(14) HOBt: 1-hydroxybenzotriazole
(15) HOCt: 6-chloro-1-hydroxybenzotriazole
(16) HOAt: 1-hydroxy-7-azabenzotriazole
(17) HOOBt: 3-hydroxy-3,4-dihydro-4-oxo-1.2.3-benzotriazine
(18) HOSu: N-hydroxysuccinimide
(19) HOPht: N-hydroxyphthalimide
(20) HONb: N-hydroxy-5-norbornane-2,3-dicarboximide
(21) Bt: 1-hydroxybenzotriazol-1-yl
(22) Ct: 1-hydroxy-6-chlorobenzotriazol-1-yl
(23) At: 1-hydroxy-7-azabenzotriazol-1-yl
(24) OBt: 3,4-dihydro-4-oxo-1.2.3-benzotriazin-3-yl
(25) Su: succinimidyl
(26) Pht: phthalimidyl
(27) Nb: 5-norbornane-2,3-dicarboximidyl
(28) DCC: N,N'-dicyclohexylcarbodiimide
(29) EDC: N-(3-dimethylaminopropyl)-N'-ethylcarbodiimide
(30) EDC.HCl: N-(3-dimethylaminopropyl)-N'-ethylcarbodiimide hydrochloride
(31) DIC: N,N'-diisopropylcarbodiimide
(32) BOP: (benzotriazol-1-yloxy)tris(dimethylamino)phosphonium hexafluorophosphate
(33) PyBOP: (benzotriazol-1-yloxy)tripyrrolidinophosphonium hexafluorophosphate
(34) PyBroP: bromotripyrrolidinophosphonium hexafluorophosphate
(35) HBTU: O-(benzotriazol-1-yl)-N,N,N',N'-tetramethyluronium hexafluorophosphate
(36) TBTU: O-(benzotriazol-1-yl)-N,N,N',N'-tetramethyluronium tetrafluoroborate
(37) HCTU: O-(6-chlorobenzotriazol-1-yl)-N,N,N',N'-tetramethyluronium hexafluorophosphate
(38) HATU: O-(7-azabenzotriazol-1-yl)-N,N,N',N'-tetramethyluronium hexafluorophosphate
(39) CDI: carbonyldiimidazole
(40) DMT-MM: 4-(4,6-dimethoxy-1,3,5-triazin-2-yl)-4-methylmorpholinium chloride
(41) AAₙ: amino acid residue (subscript n is any integer of 1 or above, which shows the order from peptide C-terminal)
(42) PG₀: protecting group of C-terminal carboxyl group of peptide
(43) PGₙ: amino-protecting group (subscript n is any integer of 1 or above, which is protecting group of amino group of AAₙ)
(44) HOE: activator
(45) E: activated ester group
(46) Gly: glycine
(47) Ala: alanine
(48) Val: valine
(49) Leu: leucine
(50) Ile: isoleucine
(51) Met: methionine
(52) Phe: phenylalanine
(53) Tyr: tyrosine
(54) Trp: tryptophan
(55) His: histidine
(56) Lys: lysine
(57) Arg: arginine
(58) Ser: serine
(59) Thr: threonine
(60) Asp aspartic acid
(61) Glu: glutamic acid
(62) Asn: asparagine
(63) Gln: glutamine
(64) Cys: cysteine
(65) Pro: proline
(66) Orn: ornithine
(67) Sar: sarcosine
(68) β-Ala: β-alanine
(69) GABA: γ-aminobutyric acid
(70) Dap: 2,3-diaminopropionic acid

Examples of the protecting group of the C-terminal carboxyl group for PGₒ include alkyl groups such as Me, Et, iPr, tBu and the like, Bzl, Fm, Trt, diphenylmethyl, 1-1-dimethylbenzyl, dimethylphenyl and the like.
Examples of the amino-protecting group for PGₙ include Boc, Z, Fmoc, Bsmoc, Alloc, Ac and the like.
The activated ester group for E means a group which is easily dissociated as "EO⁻" upon nucleophilic attack by an amino group and can produce an amide bond, and examples thereof include Bt, Ct, At, OBt, Su, Pht, Nb, pentafluorophenyl and the like.

### 1. Method of removing dibenzofulvene derivative

The method of removing dibenzofulvene derivative of the present invention comprises stirring and partitioning the reaction mixture obtained by reacting an Fmoc-protected amino acid compound with an amine for deprotection in a hydrocarbon solvent having a carbon number of 5 or above and a polar organic solvent (excluding organic amide solvents) immiscible with the hydrocarbon solvent, and removing the hydrocarbon solvent layer in which the dibenzofulvene and/or the dibenzofulvene amine adduct are/is dissolved.
By such operation, the deprotected amino acid compound unexpectedly transfers preferentially to the polar organic solvent layer and is scarcely extracted into the hydrocarbon solvent layer, and the dibenzofulvene derivative is efficiently extracted into the hydrocarbon solvent layer. Therefore, the dibenzofulvene derivative can be removed by a convenient operation alone of extraction which is suitable for large-scale reactions. Thus, a desired amino acid compound can be conveniently purified or isolated without an operation such as crystallization, chromatography and the like.

Example 9 of WO 03/018188 (patent document 1) describes, for liquid phase synthesis of peptide, a method including partitioning cyclohexane from N,N-dimethylformamide (DMF), and recovering an amino acid ester or peptide from the cyclohexane layer. However, this method differs in the constitution from the method of the present invention comprising recovering an amino acid ester or peptide from a polar solvent. In this method, moreover, (3,4,5-trioctadecyloxyphenyl)methan-1-ol, which is a soluble carrier with extremely high liposolubility, is ester bonded to the C-terminal of the peptide so as to transfer an amino acid ester or peptide to a cyclohexane layer, which is a hydrocarbon solvent. Thus, the method is completely different from the present invention also in the idea, in which a dibenzofulvene derivative is removed into a hydrocarbon solvent layer, and an amino acid ester or peptide is transferred to a polar organic solvent.

The "amino acid compound" of the "amino acid compound protected with an Fmoc group" is a compound having a primary amino group and/or a secondary amino group, as well as a carboxyl group and/or an esterified, thioesterified or amidated carboxyl group, in a molecule, and is not particularly limited as long as it is efficiently transferred to the polar organic solvent layer of the partitioned layers between hydrocarbon solvent-polar organic solvent in the present invention. Preferable examples of such amino acid compound include peptide and amino acid ester.
Here, the peptide and amino acid ester may have, besides the primary amino group or secondary amino group as the N-terminal, an amine side chain functional group (amino group, indole, guanidine etc.), and these amine side chain functional groups may be protected with a protecting group. Also, the carboxyl group at the C-terminal of peptide may or may not be protected. Furthermore, when peptide and amino acid ester have a side chain functional group other than a carboxyl group and an amino group, the side chain functional group may or may not be protected. When the carboxyl group is protected, protection of a carboxyl group and the like with a group having extremely high liposolubility and not generally employed as a protecting group for peptide, such as (3,4,5-trioctadecyloxyphenyl)methyl, is not appropriate since the amino acid ester and peptide also are likely to transfer to the hydrocarbon solvent layer. Preferable examples of the carboxyl-protecting group include alkyl having a carbon number of 1 to 6 such as methyl, ethyl, tert-butyl and the like, benzyl, p-nitrobenzyl, p-methoxybenzyl, diphenylmethyl and allyl.

In the "amino acid compound protected with an Fmoc group", at least one of a primary amino group and/or a secondary amino group and an amine side chain functional group that the amino acid compound has only need to be protected with the Fmoc group. The primary amino group and/or the secondary amino group, and the amine side chain functional group, which are not protected with an Fmoc group, may be unprotected, or may be protected with a protecting group for amino (amino-protecting group) (e.g., Boc, Z, Bsmoc, Alloc, Ac etc.) other than the Fmoc group.
As the "amino acid compound protected with an Fmoc group", preferred is a compound wherein the N-terminal amino group is protected with an Fmoc group and, when an amine side chain functional group is present, the amine side chain functional group is protected with an amino-protecting group other than the Fmoc group.

The "amine" is not particularly limited as long as it has nucleophilicity of the level capable of removing the Fmoc group, such as diethylamine, dimethylamine, pyrrolidine, piperidine, morpholine, 1,8-diazabicyclo[5.4.0]undecen-7-en (DBU) and the like.
The amine preferably has a low boiling point (boiling point: about 0 to 120°) so that it can be removed by concentration under reduced pressure, and preferable examples thereof include dimethylamine, diethylamine, pyrrolidine, piperidine and morpholine.

The dibenzofulvene is a by-product generated during removal of the Fmoc group. The dibenzofulvene amine adduct means, when the amine used for the deprotection is secondary amine, a compound wherein it is added to the dibenzofulvene. These are generally referred to a "dibenzofulvene derivative" as mentioned above.

The "removal of dibenzofulvene derivative" means removing a dibenzofulvene derivative from a reaction mixture comprising an amino acid compound obtained by deprotection and a dibenzofulvene derivative, by washing, crystallization, or other purification operations.

Examples of the "hydrocarbon solvent having a carbon number of 5 or above" include n-pentane, n-hexane, n-heptane, n-octane, decalin, naphthalene or a mixed solvent thereof, with preference given to n-heptane and n-octane. In the case of a mixed solvent, they can be mixed at any ratio. A hydrocarbon solvent having a carbon number of 4 or below is likely to be miscible with a polar solvent. While there is no particular upper limit of the carbon number, a hydrocarbon solvent having a carbon number of not more than 20 is preferable in consideration of easy handling of evaporation of the solvent and the like, and availability as a solvent.

The "polar organic solvent immiscible with a hydrocarbon solvent having a carbon number of 5 or above" can be selected without limitation from those having such property. However, the polar organic solvent does not include organic amide solvents (e.g., N,N-dimethylformamide (DMF), N,N-dimethylacetamide (DMA)) and the like. Partitioning from a hydrocarbon solvent is possible even when an organic amide solvent is used. However, since a dibenzofulvene derivative is not likely to be transferred efficiently into the hydrocarbon solvent layer, the organic amide solvent layer needs to be washed many times with a hydrocarbon solvent, which lowers efficiency (see Comparative Examples 5, 6 to be mentioned later). In addition, since organic amide solvents have comparatively high boiling point, they need to be evaporated at a high temperature, which lowers efficiency and possibly decomposes the deprotected amino acid compound. Furthermore, when an organic amide solvent is removed by washing with water, an extraction operation with ethyl acetate, diethyl ether and the like is necessary, making the steps complicated.
Examples of such polar organic solvent include acetonitrile, methanol, acetone and the like and a mixed solvent thereof, with preference given to acetonitrile and methanol. When a mixed solvent is used, the solvents can be mixed at any ratio.

The method of removing dibenzofulvene derivative of the present invention can be specifically performed by first
(1) mixing an amino acid compound protected with an Fmoc group and amine in an organic solvent to give a reaction mixture containing the amino acid compound and the dibenzofulvene derivative, and then
(2) stirring the reaction mixture in a hydrocarbon solvent and a polar organic solvent, standing the mixture still to allow partitioning, and removing the hydrocarbon solvent.

The amount of the amine to be used is generally 2 to 100 equivalents, preferably 5 to 20 equivalents, relative to the amino acid compound protected with an Fmoc group. When the amount is lower than this range, the unreacted amino acid compound protected with an Fmoc group tends to remain, and when the amount is higher than this range, removal of amine tends to be difficult.

The solvent for the deprotection reaction may be any as long as it does not inhibit the deprotection reaction, and depending on the amino acid compound protected with an Fmoc group, which is the target of deprotection, for example, DMF, N-methylpyrrolidone (NMP), ethyl acetate, acetonitrile, tetrahydrofuran (THF), chloroform and the like and a mixed solvent thereof can be mentioned. When a mixed solvent is used, the solvents can be mixed at any ratio. The amount of the solvent to be used is generally 3- to 100-fold weight, preferably 5- to 20-fold weight, relative to the amino acid compound protected with an Fmoc group.

While the reaction temperature varies depending on the amino acid compound protected with an Fmoc group, which is the target of deprotection, it is generally within the range of 0 to 40°C, preferably 5 to 30°C. The reaction time is generally 1 to 20 hr when the temperature is within the above-mentioned range.

After completion of the reaction, the reaction mixture is concentrated to give a mixture containing the amino acid compound and the dibenzofulvene derivative. When amine having a low boiling point is used, the amine can be removed by the concentration operation.
A polar organic solvent and a hydrocarbon solvent are added to the mixture thus obtained, the mixture is stirred and stood still, and the polar organic solvent and hydrocarbon solvent can be partitioned.
Alternatively, when the polar organic solvent and/or the hydrocarbon solvent of the present invention are/is used for the deprotection reaction, the polar organic solvent and/or hydrocarbon solvent are/is added as necessary without concentration, the mixture is stirred and stood still, whereby the mixture is partitioned between the polar organic solvent and the hydrocarbon solvent.
In this case, the polar organic solvent and the hydrocarbon solvent may be homogenized by heating, and then cooled for partitioning. However, since the operation is complicated and the effect of the present invention can be sufficiently afforded by stirring and standing still alone, the operation accompanying heating and cooling is not necessary.

Water may be further added to the polar organic solvent to improve partitioning performance from the hydrocarbon solvent, and to improve dibenzofulvene derivative removal rate and extraction rate of the object product into the polar organic solvent layer. The content of water is preferably 1 to 50%(v/v) relative to the polar organic solvent. When the content of water is higher than this range, the extraction efficiency of the amino acid compound protected with an Fmoc group may decrease.
While the amount of the polar organic solvent to be used is not particularly limited as long as it does not impair the effect of the present invention, it can be appropriately selected from the range setting the concentration of the amino acid compound protected with an Fmoc group to 0.5 to 30%(w/v) relative to the polar organic solvent.

While the amount of the hydrocarbon solvent to be used is not particularly limited as long as it does not impair the effect of the present invention, it can be appropriately selected from the range setting the concentration of the dibenzofulvene derivative to 0.2 to 3%(w/v) relative to the hydrocarbon solvent.

While the (polar organic solvent)/(hydrocarbon solvent) ratio (v/v) is not particularly limited as long as it does not impair the effect of the present invention, it is preferably 1/10 to 1/0.2, more preferably 1/3 to 1/0.5.

After stirring and standing still, the hydrocarbon solvent layer is removed to remove the dibenzofulvene derivative. The hydrocarbon solvent can be further added to the polar organic solvent layer, followed by stirring and standing still of the mixture, and removal of the hydrocarbon solvent layer. This operation can be repeated plural times, whereby the dibenzofulvene derivative remaining in the polar organic solvent layer can be further removed.
By concentrating the polar organic solvent layer, a crude product containing the amino acid compound free of dibenzofulvene derivative can be obtained. The amino acid compound can sometimes be isolated at this stage, but when impurities (e.g., amine used for deprotection etc.) other than the dibenzofulvene derivative are contained, a desired amino acid compound can be isolated by combining isolation and purification operations known per se (washing with water, washing with basic or acidic aqueous solution etc.).

### 2. Peptide liquid phase synthesis method

When the amino acid compound is peptide or an amino acid ester, the method of removing dibenzofulvene derivative of the present invention can be preferably applied to a liquid phase synthesis method of peptide. An embodiment wherein the method of removing dibenzofulvene derivative of the present invention is applied to a liquid phase synthesis method of peptide (hereinafter to be referred to as the "liquid phase synthesis method of peptide of the present invention" in the present specification) is explained in the following. Needless to say, the present invention is not limited thereto.

### 2-1. Explanation of terms

The meanings of the terms used for the liquid phase synthesis method of peptide of the present invention are now explained.
In the liquid phase synthesis method of peptide of the present invention, the deprotection of the "peptide or amino acid ester protected with an Fmoc group" is not particularly limited as long as it is a deprotection of an Fmoc group included in the liquid phase synthesis method of peptide. For example, it may be N-terminal deprotection step as described below or a deprotection of a side chain amino group in the final deprotection. It is preferably a deprotection to produce an amino group to be the object of coupling in the next step in the peptide elongation reaction to be described below.
When an amino acid is shown by "H-AA-OH" in the liquid phase synthesis method of peptide of the present invention, the left side is an amino group and the right side is a carboxyl group, and the amino group and the carboxyl group are independently not protected.
In this case, for example, when the carboxyl group is protected, the amino acid is shown by "H-AA-OPG₀", and when the amino group is protected, it is shown by "PGₙ-AA-OH".
When the carboxyl group of amino acid with a protected amino group becomes active ester, the amino acid is shown by "PGₙ-AA-OE".
A symmetric acid anhydride of PGₙ-AA-OH is shown by "(PGₙ-AA)₂-O".
When amino acid has a protected side chain functional group, it is shown by "H-AA(PG)-OH" (PG is a protecting group of the side chain functional group).

When peptide is shown by "H-AA_{n'}-AA_{n'-1}- ··· -AA₁-OH" (subscript n' is any integer of two or above) in the liquid phase synthesis method of peptide of the present invention, it means that the left side is N-terminal, the right side is C-terminal, and the peptide contains amino acid residues in the number of n' with unprotected N-terminal and unprotected C-terminal. Here, the N-terminal is not limited to an α-amino group of the amino acid residue, and also includes a side chain amino group when peptide elongation is performed via the side chain amino group (e.g., ε-amino group Lys), hereinafter the same.
In this case, for example, when the C-terminal is protected, it is shown by "H-AAₙ,-AA_{n'-1}- ··· -AA₁-OPG₀", and further, when the N-terminal is protected, it is shown by "PG_{n'}-AA_{n'}-AA_{n'-1}- ··· -AA₁-OPG₀".

The amino acid residue to be a constituent unit of peptide synthesized by the liquid phase synthesis method of peptide of the present invention nonlimitatively includes natural amino acids and nonnatural amino acids, as well as L forms and racemates thereof.
Examples of the natural amino acid include Gly, Ala, Val, Leu, Ile, Ser, Thr, Asn, Gln, Asp, Glu, Lys, Arg, Cys, Met, Phe, Tyr, Trp, His, Pro, Orn, Sar, β-Ala, GABA and the like.
Examples of the nonnatural amino acid include Dap and the like.
In addition, when the amino acid has a functional group in the side chain, the functional group of the amino acid can be protected with a protecting group. Examples of the amino acid with protected side chain include γ-Bzl-Glu or β-Bzl-Asp, wherein a carboxyl group at the γ- position of Glu or β-position of Asp is protected with a benzyl group; γ-tBu-Glu or β-tBu-Asp, wherein a carboxyl group at the γ-position of Glu or β-position of Asp is protected with a tert-butyl group; ε-Z-Lys, ε-Boc-Lys, ε-iPr-E-Boc-Lys, wherein a ε-amino group of Lys is protected; S-phenylcarbamoyl-Cys wherein a SH group of Cys is protected with a phenylcarbamoyl group; S-Trt-Cys wherein an SH group of Cys is protected with a trityl group; a derivative wherein oxygen of a hydroxyl group of Tyr and Ser is protected with Bzl and the like.

In the liquid phase synthesis method of peptide of the present invention, the "N-protected amino acid" means an amino acid wherein the amino group is protected and the carboxyl group is unprotected, which is shown by "PGₙ-AA-OH" according to the above-mentioned notation.
In the liquid phase synthesis method of peptide of the present invention, the "N-protected amino acid activated ester" means an amino acid wherein the amino group is protected and the carboxyl group becomes active ester with E, and according to the above-mentioned notation, it is shown by "PGₙ-AA-OE".
In the N-protected amino acid activated ester that can be isolated, E is pentafluorophenyl, Su or Nb, and other N-protected amino acid activated esters are produced in a reaction system by reacting N-protected amino acid with a condensing agent (e.g., EDC) and an activator (e.g., HOBt).

In the liquid phase synthesis method of peptide of the present invention, the "N-Fmoc amino acid" means an amino acid residue wherein the amino group is protected with Fmoc, and the carboxyl group is unprotected, which is shown by "Fmoc-AA-OH" according to the above-mentioned notation.
In the liquid phase synthesis method of peptide of the present invention, the "N-Fmoc amino acid activated ester" means any amino acid residue wherein an amino group is protected with Fmoc and a carboxyl group becomes active ester with E, which is shown by "Fmoc-AA-OE" according to the above-mentioned notation.
In the N-Fmoc amino acid activated ester that can be isolated, E is pentafluorophenyl, Su or Nb. Other N-Fmoc amino acid activated esters are produced in a reaction system by reacting N-Fmoc amino acid with a condensing agent (e.g., EDC) and an activator (e.g., HOBt).

In the liquid phase synthesis method of peptide of the present invention, the "C-protected peptide" means a peptide containing amino acid residues in any number, wherein the C-terminal is protected and the N-terminal is not protected. According to the above-mentioned notation, it is shown by "H-AA_{n'}-AA_{n'-1}- ··· -AA₁-OPG₀" (n' is an integer of two or above).
In the liquid phase synthesis method of peptide of the present invention, the "C-protected amino acid" means an amino acid wherein the carboxyl group is protected and the amino group is not protected. According to the above-mentioned notation, it is shown by "H-AA-OPG₀".
In the liquid phase synthesis method of peptide of the present invention, the "N,C-deprotected peptide" means a peptide containing amino acid residues in any number, wherein both the N-terminal and the C-terminal are protected.
According to the above-mentioned notation, it is shown by "PG_{n'}-AA_{n'}-AA_{n'-1}- ··· -AA₁-OPG₀" (n' is an integer of two or above). In addition, for example, N,C-deprotected peptide wherein the N-terminal is protected with Fmoc and the C-terminal is protected is shown by "N-Fmoc-C-protected peptide".
In the liquid phase synthesis method of peptide of the present invention, the "intermediate peptide" is a synthesis intermediate peptide obtained in each step in the liquid phase synthesis of peptide, which contains amino acid residues in a number less than that in the final object peptide. Preferable intermediate peptide is C-protected peptide obtained after deprotection of N-terminal as mentioned below

In the liquid phase synthesis method of peptide of the present invention, examples of the "condensing agent" include DCC, EDC (including hydrochloride and free form), DIC, BOP, PyBOP, PyBroP, HBTU, HCTU, TBTU, HATU, CDI, DMT-MM and the like.
In the liquid phase synthesis method of peptide of the present invention, the "activator" is a reagent that leads a carboxyl group to activated ester, symmetric acid anhydride and the like in the co-presence of a condensing agent, and facilitates amide bond formation. It is shown by "HOE". Specific examples include HOBt, HOCt, HOAt, HOOBt, HOSu, HOPht, HONB, pentafluorophenol and the like.

In the liquid phase synthesis method of peptide of the present invention, the "one-pot synthesis" means, in the liquid phase synthesis method of peptide, synthesis up to objective peptide without taking out an intermediate peptide obtained in each step from the reaction vessel.

### 2-2. Peptide liquid phase synthesis method

The peptide finally synthesized by the liquid phase synthesis method of peptide of the present invention is not particularly limited, and can be preferably utilized for the synthesis of synthetic pharmaceutical peptide, cosmetic, electronic material (organic EL etc.), food and the like.
While the number of the constituent amino acid residues of the peptide is not particularly limited, it is preferably about 2 to 20 residues found in general synthetic peptide.
In addition, the liquid phase synthesis method of peptide of the present invention is suitable for the liquid phase synthesis method of peptide and the like using C-protected peptide (e.g., peptide including ε-Boc-Lys etc.) wherein amino acid side chain functional group and/or C-terminal protecting group is protected with a protecting group which is removed with an acid.

The "liquid phase synthesis method of peptide" means that it is not a solid phase method, and the method of the present invention includes not only a case where all reagents are dissolved in a solvent, but also what is called a heterogeneous reaction where reagents are entirely or partly undissolved but suspended and the like in a solvent.

For the liquid phase synthesis method of peptide, a general method conventionally used in the peptide synthesis chemistry can be employed without particular limitation.
Specifically, it is a method shown in the following scheme, in other words, a method including repeats of one cycle reaction (hereinafter to be referred to as "peptide elongation reaction" in the present specification) consisting of a step of obtaining N,C-deprotected peptide (PPₙ₊₁) wherein one amino acid residue is elongated by
(1) condensing C-protected peptide (P_{n'}) wherein n' is any integer of two or above, and means that peptide contains amino acid residues in the number of n', hereinafter the same) or, in the first peptide elongation, C-protected amino acid (A₁) (hereinafter to be collectively referred to as "C-protected peptide (Pₙ) or the other" (n is any integer of one or more and, when n is 1, it means C-protected amino acid (A₁), hereinafter the same) in the present specification) with N-protected amino acid (PAₙ₊₁) in the presence of a condensing agent (and preferably an activator), or
(2) condensing C-protected peptide (Pₙ) or the other with N-protected amino acid activated ester (PAEₙ₊₁) (hereinafter to be referred to as "coupling step (1)" and "coupling step (2)", respectively, in the present specification), and a step of obtaining C-protected peptide (Pₙ₊₁) by removing an amino-protecting group of the obtained N,C-deprotected peptide (PPₙ₊₁) (hereinafter to be referred to as "N-terminal deprotection step" in the present specification). In the final step, the object peptide (P) can be obtained by removing the carboxy-protecting group of C-protected peptide (Pₘ) and a protecting group in case of a protected side chain functional group (hereinafter to be referred to as the "final deprotection step" in the present specification).
   In the peptide synthesis method in the present invention, the nth peptide elongation reaction is indicated as "peptide elongation reaction (n)", and respective steps constituting the peptide elongation reaction (n) are indicated as "coupling step (1-n)", "coupling step (2-n)" and "N-terminal deprotection step (n)".

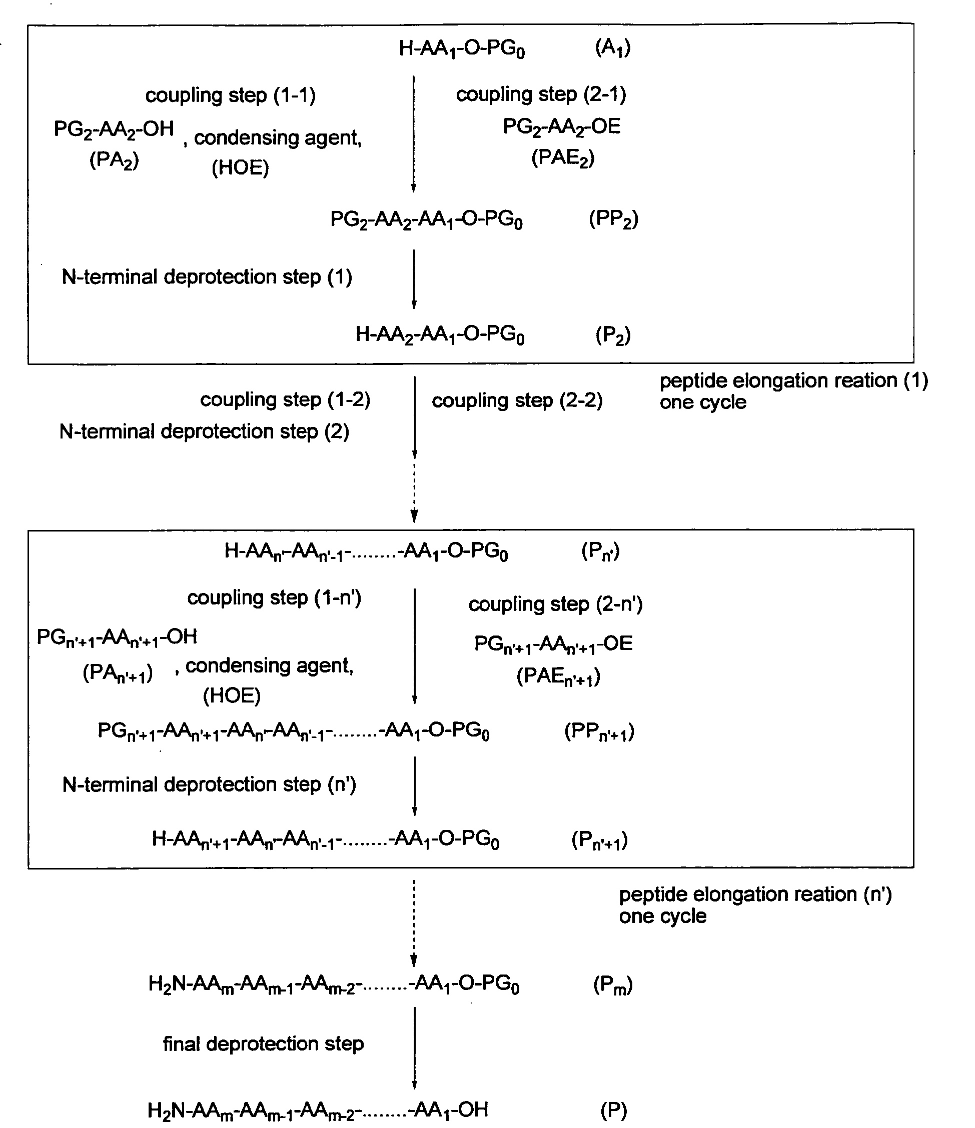

wherein m is the number of amino acid residues of the objective peptide, and other symbols are as defined above.
The present invention is **characterized in that,** in at least once of a series of peptide elongation reactions, coupling step (1) is performed using N-Fmoc amino acid or coupling step (2) is performed using N-Fmoc amino acid activated ester to give N-Fmoc-C-protected peptide, the N-Fmoc-C-protected peptide is deprotected by reaction with an amine in the subsequent N-terminal deprotection step to give a mixture containing C-protected peptide (Pₙ₊₁) and a dibenzofulvene derivative, the mixture is partitioned between a polar organic solvent and a hydrocarbon solvent in the subsequent workup, and the hydrocarbon solvent layer containing dibenzofulvene derivative dissolved therein is removed (hereinafter to be referred to as "peptide elongation reaction in the present invention", and the nth peptide elongation reaction is referred to as "peptide elongation reaction (n) in the present invention").
In the liquid phase synthesis method of peptide of the present invention, the peptide elongation reaction in the present invention only needs to be contained at least once. However, all steps are preferably performed in the peptide elongation reaction in the present invention, whereby the objective peptide can be synthesized in one-pot.
The scheme of the peptide elongation reaction (n) in the present invention is shown below.

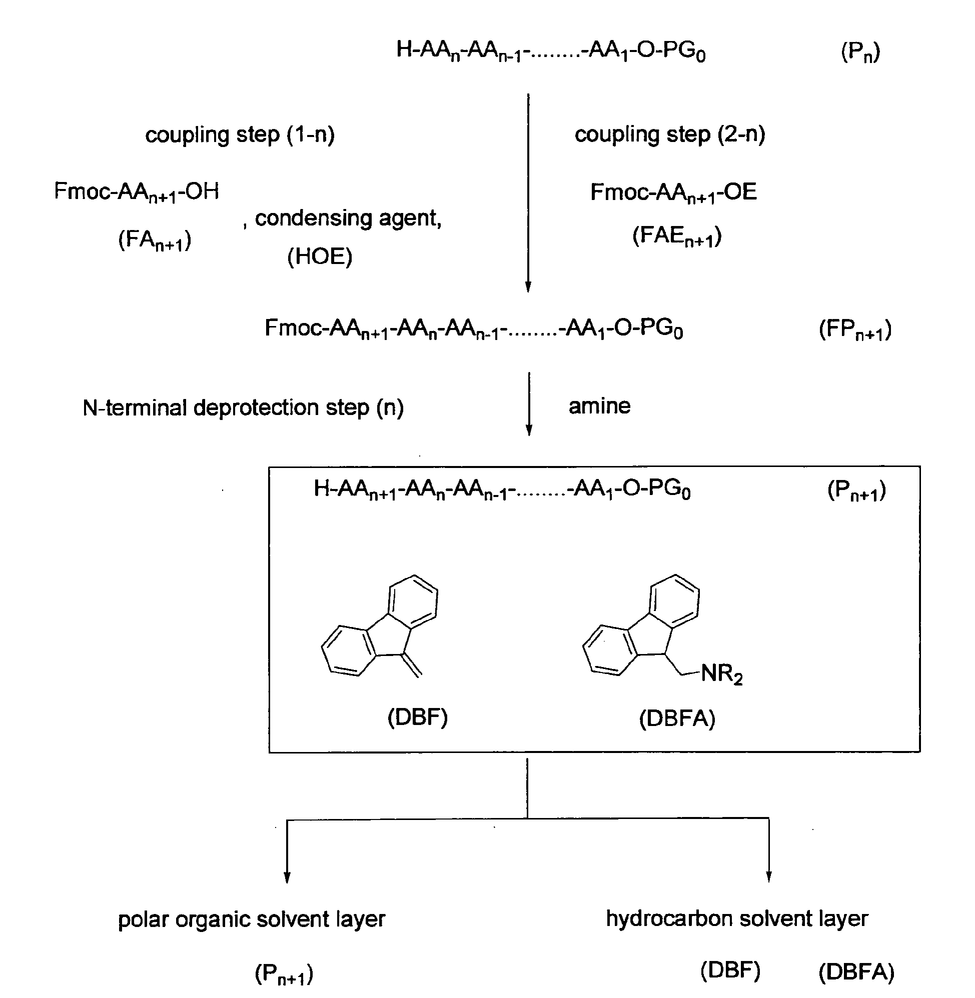

wherein DBF is dibenzofulvene, DBFA is a dibenzofulvene amine adduct, NR₂ corresponds to amine used for deprotection (in case of secondary amine), and other symbols are as defined above.

By partitioning a mixture of C-protected peptide (Pₙ₊₁) and a dibenzofulvene derivative between a polar organic solvent and a hydrocarbon solvent, C-protected peptide (Pₙ₊₁) unexpectedly transfers preferentially to the polar organic solvent layer and is scarcely extracted into the hydrocarbon solvent layer, and the dibenzofulvene derivative is efficiently extracted into the hydrocarbon solvent layer. Therefore, the dibenzofulvene derivative can be removed by a convenient operation alone of extraction which is suitable for large-scale reactions. Thus, the obtained C-protected peptide (Pₙ₊₁) can be provided to the next peptide elongation reaction (n+1) without an isolation and purification operation such as crystallization, chromatography and the like, thus leading to the one-pot synthesis.

The liquid phase synthesis method of peptide of the present invention is explained in detail in the following.

### 2-2-1. Coupling step (1)

In coupling step (1) in the peptide elongation reaction in the present invention, for example, N-Fmoc amino acid (FAₙ₊₁). C-protected peptide (Pₙ) or the other, and a condensing agent are mixed (preferably with an activator) in a solvent to give N-Fmoc-C-protected peptide (FPₙ₊₁) wherein one amino acid residue is elongated. While the order of addition is not particularly limited, when the C-protected peptide (Pₙ) or the other is obtained by peptide elongation reaction (n-1) before this one, N-Fmoc amino acid (FAₙ₊₁) and a condensing agent can be added to a solution of the C-protected peptide (Pₙ) or the other in a reaction vessel.

The amount of N-Fmoc amino acid (FAₙ₊₁) to be used is generally 0.9 to 4.0 equivalents, preferably 1.0 to 1.5 equivalents, relative to the C-protected peptide (Pₙ) or the other. When the amount is smaller than this range, unreacted C-protected peptide (Pₙ) or the other tends to remain, and when the amount is higher, excess N-Fmoc amino acid (FAₙ₊₁) cannot be removed easily.

When C-protected peptide (Pₙ) or the other is used as acid addition salt, a base is added for neutralization. Examples of the base include triethylamine, diisopropylethylamine, pyridine, N-methylmorpholine and the like. The amount of the base to be used is generally 0.5 to 2.0 equivalents, preferably 1.0 to 1.5 equivalents, relative to the C-protected peptide (Pₙ) or the other. When the amount of the base to be used is smaller than this range, neutralization becomes insufficient and the reaction does not proceed smoothly.

As the condensing agent, those exemplified above can be used without particularly limitation. Examples thereof include EDC (free form or hydrochloride), DIC, DCC, PyBOP, HBTU, HCTU and DMT-MM. EDC is preferable since the residual condensing agent and decomposed condensing agent can be removed with ease by washing. The amount of the condensing agent to be used is generally 0.8 to 4.0 equivalents, preferably 1.0 to 1.5 equivalents, relative to N-Fmoc amino acid (FAₙ₊₁).

In coupling step (1), an activator is preferably added to promote the reaction and suppress side reactions such as racemization and the like. When an activator is present, activated ester of N-protected amino acid, and the like are temporarily produced in the reaction system.
As the activator, those exemplified above can be used without particularly limitation, and HOBt, HOOBt, HOCt, HOAt, HONb, HOSu and the like are preferable. The amount of the activator to be used is generally 0 to 4.0 equivalents, preferably 0.1 to 1.5 equivalents, relative to N-Fmoc amino acid (FAₙ₊₁).

The solvent may be any as long as it does not inhibit the reaction and, for example, N,N-dimethylformamide (DMF), N-methylpyrrolidone (NMP), ethyl acetate, tetrahydrofuran (THF), acetonitrile, chloroform, methylene chloride and the like or a mixed solvent thereof can be mentioned, with preference given to ethyl acetate and DMF. The amount of the solvent to be used is generally 3- to 100-fold weight, preferably 5- to 20-fold weight, relative to the C-protected peptide (Pₙ) or the other.

The reaction temperature is generally within the range of -20°C to 40°C, preferably 0°C to 30°C. The reaction time is generally 0.5 to 30 hr within the above-mentioned temperature range.

Since the workup after completion of coupling step (1) reaction is the same as for coupling step (2), it is explained all together in the below-mentioned 2-2-3.

### 2-2-2. Coupling step (2)

In coupling step (2) in the peptide elongation reaction in the present invention, for example, N-Fmoc amino acid activated ester (FAEₙ₊₁) and the C-protected peptide (Pₙ) or the other are mixed in a solvent to give N-Fmoc-C-protected peptide (FPₙ₊₁). While the order of addition is not particularly limited, when the C-protected peptide (Pₙ) or the other is obtained by peptide elongation reaction (n-1) before this one, N-Fmoc amino acid activated ester (FAEₙ₊₁) can be added to a solution of C-protected peptide (Pₙ) or the other in a reaction vessel.

The amount of N-Fmoc amino acid activated ester (FAEₙ₊₁) to be used is the same as N-Fmoc amino acid (FAₙ₊₁) in coupling step (1).
In addition, other reaction conditions such as base, solvent and amounts thereof to be used, reaction temperature, reaction time and the like are the same as those for coupling step (1).

### 2-2-3. Workup of coupling steps (1) and (2)

After the completion of the reactions of coupling steps (1) and (2), solid nucleophile removing reagents such as thiol group-supported silica gel and the like (e.g., SH silica, NH silica (manufactured by Fuji Silysia Chemical Ltd.) etc.) may be added, and the mixture is stirred and filtered to remove residues and byproducts in the reaction mixture that can be condensed with amine components, such as N-Fmoc amino acid activated ester (FAEₙ₊₁), isourea ester of N-Fmoc amino acid, symmetric acid anhydride of N-Fmoc amino acid and the like. In addition, activated ester may be positively deactivated in the washing step by washing with weak alkaline aqueous solution such as sodium carbonate and the like.

In the workup in coupling steps (1) and (2), washing with acidic aqueous solution and/or washing with basic aqueous solution is preferably performed. The washing with acidic aqueous solution can remove C-protected peptide, residual condensing agent and a decomposed product thereof, a base and the like into the aqueous layer. The washing with basic aqueous solution can remove activator, residual N-Fmoc amino acid and the like into the aqueous layer.

The washing with acidic aqueous solution is performed by, for example, stirring the reaction mixture with dilute aqueous hydrochloric acid solution (e.g., 1N aqueous hydrochloric acid solution), an aqueous solution of sulfuric acid, formic acid, citric acid, phosphoric acid and the like, partitioning and removing the aqueous layer.

The washing with basic aqueous solution is performed by, for example, stirring the reaction mixture with an aqueous solution such as aqueous sodium hydrogen carbonate solution (e.g., 5% aqueous sodium hydrogen carbonate solution), aqueous sodium carbonate solution, aqueous potassium carbonate solution and the like, partitioning and removing the aqueous layer.

N-Fmoc-C-protected peptide (FPₙ₊₁) can be obtained by further washing with water as necessary and concentrating the organic layer, which can be subjected to the N-terminal deprotection step without taking out from the vessel. In addition, it may be used without concentration for the N-terminal deprotection step as a solution of N-Fmoc-C-protected peptide (FPₙ₊₁).

When the liquid phase synthesis method of peptide of the present invention includes a coupling step using an amine-protecting group other than Fmoc, it can also be performed in the same manner as in the above.

### 2-2-4. N-terminal deprotection step

In the N-terminal deprotection step of the peptide elongation reaction in the present invention, C-protected peptide (Pₙ₊₁) can be obtained by reacting N-Fmoc-C-protected peptide (FPₙ₊₁) with an amine in a solvent. Specifically, the amine can be added to a solution of N-Fmoc-C-protected peptide (FPₙ₊₁) obtained in the coupling step.
A mixture of C-protected peptide (Pₙ₊₁) and a dibenzofulvene derivative obtained by the deprotection reaction is stirred in a hydrocarbon solvent having a carbon number of 5 or above and a polar organic solvent (excluding organic amide solvents) immiscible with the hydrocarbon solvent, partitioned and the hydrocarbon solvent layer is removed, whereby the dibenzofulvene derivative can be removed.
In the first elongation reaction, C-protected peptide (Pₙ) or the other is C-protected amino acid. The C-protected amino acid can be prepared as follows. That is, the carboxyl group of N-Fmoc amino acid is esterified by a conventional method to give N-Fmoc-C-protected amino acid. In the same manner as above, the obtained N-Fmoc-C-protected amino acid is reacted with an amine in a solvent to give C-protected amino acid (amino acid ester). In the same manner as above, a mixture of the C-protected amino acid and a dibenzofulvene derivative obtained by the deprotection reaction is stirred in a hydrocarbon solvent having a carbon number of 5 or above and a polar organic solvent (excluding organic amide solvents) immiscible with the hydrocarbon solvent, partitioned and the hydrocarbon solvent layer is remove, whereby the dibenzofulvene derivative can be removed. The deprotection of the Fmoc group of the N-Fmoc-C-protected amino acid is also included in the N-terminal deprotection step here.

The N-terminal deprotection step in the liquid phase synthesis method of peptide of the present invention can be performed in the same manner by replacing an "amino acid compound protected with an Fmoc group" with "N-Fmoc-C-protected peptide (FPₙ₊₁)" or "N-Fmoc-C-protected amino acid", and replacing "amino acid compound" with "C-protected peptide (Pₙ₊₁)" or "C-protected amino acid" in the "method of removing dibenzofulvene derivative" in the above-mentioned 1.
The solution of C-protected peptide (Pₙ₊₁) isolated in the N-terminal deprotection step can be directly used with or without concentration for the next peptide elongation reaction.

When the peptide synthesis in the present invention includes an N-terminal deprotection step using an N-terminal protecting group other than Fmoc, it can be performed according to a general N-terminal deprotection method according to the kind of the amino-protecting group conventionally used for the peptide synthesis chemistry.
As mentioned above, according to the method of the present invention, dibenzofulvene and/or a dibenzofulvene amine adduct can be conveniently removed from the reaction mixture obtained by reacting an amino acid compound protected with an Fmoc group with an amine for deprotection in the N-terminal deprotection step. Therefore, the next condensation step, i.e., the next peptide elongation reaction, in the liquid phase synthesis method of peptide can be performed without taking intermediate peptide from the reaction vessel. In other words, the next condensation step can be performed without isolating the obtained intermediate peptide as a solid by crystallization and the like. Thus, according to the method of the present invention, the object final peptide can be synthesized in one-pot by the liquid phase synthesis method of peptide.

### 2-2-5. Final deprotection step

In the peptide synthesis in the present invention, the objective peptide (P) can be obtained by removing PG₀ and side chain protecting group from C-protected peptide (Pₘ) constructed up to the objective peptide.
For the final deprotection step, a deprotection method known per se can be employed without particular limitation according to the kind of PG₀ or side chain protecting group.
For example, in the case of a lower alkyl group such as Me, Et and the like, the reaction can be performed with a base such as sodium hydroxide, potassium hydroxide and the like in a solvent such as water, aqueous organic solvent and the like at -20°C to 40°C for 0.5 to 10 hr.
tBu can be removed by reaction with an acid such as trifluoroacetic acid, hydrochloric acid, methanesulfonic acid, tosylic acid, formic acid and the like in a solvent such as chloroform, methylene chloride, ethyl acetate, dioxane and the like at -20°C to 40°C for 0.5 to 10 hr.
Bzl can be removed by hydrogenation reaction using a catalyst such as palladium carbon and the like in a solvent such as methanol, DMF, acetic acid and the like at 0°C to 40°C for 0.5 to 100 hr, or by reaction with a strong acid such as hydrogen fluoride, trifluoromethanesulfonic acid and the like at -20°C to 40°C for 0.5 to 10 hr.
In the case of an alloc group, the reaction can be performed with a homogeneous zero-valent palladium catalyst such as tetrakistriphenylphosphinepalladium and the like. The homogeneous zero-valent palladium catalyst is used in 0.01 to 1.0 equivalent, preferably 0.05 to 0.5 equivalent.
When the side chain amino group protected with an Fmoc group is deprotected in the final deprotection step, the method of removing dibenzofulvene derivative of the present invention can also be applied in the workup in the same manner as in the N-terminal deprotection step.

Peptide (P) synthesized by the method of the present invention can be isolated and purified by a method conventionally used in the peptide chemistry. For example, peptide (P) can be isolated and purified by extraction, washing, crystallization, chromatography and the like of the reaction mixture in the workup of the C-terminal deprotection step.

### Examples

The present invention is explained in more detail in the following by referring to Examples, which are not to be construed as limitative.
HPLC analysis was performed under the following conditions.
column: YMC-Pack 150 mm x 4.6 mm 12 nm 5 µm
mobile phase: 0.1°s TFA aq./0.1% TFA-MeCN

### Reference Example 1

H-Ala-OtBu.HCl (1.00 g, 5.50 mmol) was dissolved in DMF (10 ml), and triethylamine (0.80 ml, 5.78 mmol) was added.
Fmoc-Phe-OH (2.24 g, 5.78 mmol) and HOBt (0.15 g, 1.1 mmol) were added, EDC.HCl (1.21 g, 6.06 mmol) was added under ice-cooling, and the mixture was stirred at room temperature for 3 hr. The reaction mixture was washed successively with 5% aqueous sodium hydrogen carbonate solution, 1N aqueous hydrochloric acid and saturated brine, and the organic layer was concentrated. Hexane was added to the residue, and the precipitate was collected by filtration and dried under reduced pressure to give Fmoc-Phe-Ala-OtBu (2.80 g, 5.44 mmol).

### Reference Example 2

H-Cys(Trt)-OtBu.HCl (6.11 g, 13.40 mmol) was partitioned between AcOEt (65 ml) and 10% aqueous sodium carbonate solution (65 ml), followed by washing with saturated brine. Fmoc-Dap(Alloc)-OH (5.50 g, 13.40 mmol) and HOBt (1.81 g, 13.40 mmol) were added to the obtained organic layer, EDC.HCl (2.83 g, 14.74 mmol) was added under ice-cooling, and the mixture was stirred at room temperature for 3 hr. AcOEt (65 ml) was added to the reaction mixture, the mixture was washed successively with 5% aqueous sodium hydrogen carbonate solution, 1N aqueous hydrochloric acid and saturated brine, and the organic layer was concentrated. Hexane was added to the residue, and the precipitate was collected by filtration and dried under reduced pressure to give Fmoc-Dap(Alloc)-Cys(Trt)-OtBu (10.35 g, 12.75 mmol).

### Example 1

Fmoc-Phe-Ala-OtBu (0.50 g, 0.97 mmol) was dissolved in acetonitrile (10 ml), diethylamine (1.02 ml, 9.72 mmol) was added, and the mixture was stirred for 2 hr. The reaction mixture was concentrated to dryness, dissolved in 90% aqueous acetonitrile (8 ml), and washed 3 times with heptane (8 ml). As a result of the HPLC analysis, the objective product, H-Phe-Ala-OtBu, was not detected in the heptane layer, and the removal rate of the fulvene derivative from the reaction mixture into the heptane layer was 93%.

### Example 2

Fmoc-Dap(Alloc)-Cys(Trt)-OtBu (0.70 g, 0.86 mmol) was dissolved in acetonitrile (12 ml), diethylamine (0.64 ml) was added, and the mixture was stirred at room temperature for 2 hr. The reaction mixture was concentrated under reduced pressure, the residue was dissolved in acetonitrile (12 ml), and the mixture was washed 3 times with heptane (7 ml). The acetonitrile layer was concentrated, and the residue was analyzed by HPLC. H-Dap(Alloc)-Cys(Trt)-OtBu (0.50 g, 0.85 mmol) was obtained. As a result of the HPLC analysis, the removal rate of the fulvene derivative from the reaction mixture into the heptane layer was 81%. The objective product was not detected in the heptane layer.

### Example 3

EYtioc-Phe-Ala-OtBu (0.25 g, 0.49 mmol) was dissolved in acetonitrile (4 ml), diethylamine (1.02 ml) was added, and the mixture was stirred at room temperature for 2 hr. The reaction mixture was concentrated under reduced pressure, and the residue was dissolved in 90% aqueous acetonitrile (3 ml) and washed 3 times with hexane (4 ml). As a result of the HPLC analysis, H-Phe-Ala-OtBu was not detected in the hexane layer, and the removal rate of the fulvene derivative from the reaction mixture into the hexane layer was 85%.

### Example 4

In the same manner as in the above-mentioned Example 3, the operation was performed using octane (4 ml) instead of hexane as a washing solvent. As a result of the HPLC analysis, H-Phe-Ala-OtBu was not detected in the octane layer, and the removal rate of the fulvene derivative from the reaction mixture into the octane layer was 95%.

### Example 5

In the same manner as in the above-mentioned Example 3, the operation was performed using decalin (4 ml) instead of hexane as a washing solvent. As a result of the HPLC analysis, H-Phe-Ala-OtBu was not detected in the decalin layer, and the removal rate of the fulvene derivative from the reaction mixture into the octane layer was 93%.

### Example 6

In the same manner as in the above-mentioned Example 3, the operation was performed using heptane-methanol (4 ml) instead of hexane-90% aqueous acetonitrile. As a result of the HPLC analysis, H-Phe-Ala-OtBu was not detected in the heptane layer, and the removal rate of the fulvene derivative from the reaction mixture into the octane layer was 86%.

### Example 7

H-Ala-OtBu.HCl (0.50 g, 2.25 mmol) was dissolved in DMF (5 ml), and triethylamine (0.40 ml, 2.89 mmol) was added. Fmoc-Phe-OH (1.12 g, 2.89 mmol) and HOBt (0.75 g, 0.55 mmol) were added, EDC.HCl (0.60 g, 3.03 mmol) was added under ice-cooling, and the mixture was stirred at room temperature for 3 hr. SH silica (0.70 g) was added to the reaction mixture, and the mixture was stirred for 30 min and filtered. Ethyl acetate (10 ml) was added to the filtrate, the mixture was washed successively with 5% aqueous sodium hydrogen carbonate solution, 1N aqueous hydrochloric acid and saturated brine, and the organic layer was concentrated. Acetonitrile (10 ml) was added to the residue, diethylamine (2 ml) was added, and the mixture was stirred at room temperature. The reaction mixture was washed 3 times with heptane (15 ml). As a result of the HPLC analysis, H-Phe-Ala-OtBu was not detected in the heptane layer, and the removal rate of the fulvene derivative from the reaction mixture was 93%.
HOBt (0.04 g, 0.27 mmol) and Fmoc-Ser(tBu)-OH (1.11 g, 2.89 mmol) were added to the obtained acetonitrile layer, HCl.EDC (0.61 g, 3.03 mmol) was added under ice-cooling, and the mixture was stirred for 3 hr. SH silica (0.70 g) was added to the reaction mixture, and the mixture was stirred for 30 min and filtered. Ethyl acetate (30 ml) was added to the filtrate, and the mixture was washed successively with 5% aqueous sodium hydrogen carbonate solution, 1N aqueous hydrochloric acid and saturated brine. The organic layer was concentrated to give Fmoc-Ser(tBu)-Phe-Ala-OtBu (1.32 g, 2.00 mmol).

### Example 8

Fmoc-Dap(Alloc)-Cys(Trt)-OtBu (11.78 g, 1.45 mmol) was dissolved in acetonitrile (120 ml), diethylamine (15.1 ml) was added, and the mixture was stirred at room temperature for 2 hr. The reaction mixture was concentrated under reduced pressure, ethyl acetate (30 ml) was added, and the mixture was concentrated again. The residue was dissolved in 90% acetonitrile (180 ml), and the mixture was washed 3 times with heptane (180 ml). As a result of the HPLC analysis, the removal rate of the fulvene derivative from the reaction mixture into the heptane layer was 86%, and the objective peptide was not detected in the heptane layer. The obtained acetonitrile layer was concentrated, ethyl acetate (180 ml) was added, and the mixture was washed successively with 10% aqueous sodium carbonate solution and saturated brine, and dried over sodium sulfate.
HOBt (0.20 g, 1.41 mmol) and Fmoc-Asp(OtBu)-OH (5.97 g, 1.45 mmol) were dissolved in the organic layer, EDC.HCl (3.06 g, 1.60 mmol) was added under ice-cooling, and the mixture was stirred for 3 hr. The reaction mixture was washed successively with 5% aqueous sodium hydrogen carbonate solution, 1N aqueous hydrochloric acid and saturated brine, and the organic layer was concentrated to give Fmoc-Asp(OtBu)-Dap(Alloc)-Cys(Trt)-OtBu (13.3 g, 1.35 mmol).

### Comparative Example 1

Fmoc-Phe-Ala-OtBu (300 mg, 0.58 mmol) was dissolved in acetonitrile (7 ml), diethylamine (0.61 ml, 5.80 mmol) was added, and the mixture was stirred for 2 hr. The reaction mixture was concentrated to dryness, and hexane was added to the residue. However, precipitation did not occur and an oily substance was obtained.

### Comparative Example 2

Fmoc-Dap(Alloc)-Cys(Trt)-OtBu (1.56 g, 1.92 mmol) was dissolved in acetonitrile (16 ml), diethylamine (1.95 ml, 19.2 mmol) was added, and the mixture was stirred at room temperature for 2 hr. The reaction mixture was concentrated under reduced pressure, and the residual oily substance was decanted twice with hexane (15 ml). Hexane was further added, and the mixture was stirred. The precipitate was collected by filtration and dried under reduced pressure to give H-Dap(Alloc)-Cys(Trt)-OtBu (1.02 g, 1.73 mmol). As a result of the HPLC analysis, the removal rate of the dibenzofulvene (DBF) from the reaction mixture into the hexane mother liquor was 83%.

### Comparative Example 3

In the same manner as in Example 3, the operation was performed except that water (4 ml) and heptane (4 ml) were used as extraction and washing solvents instead of 90% aqueous acetonitrile-hexane. As a result, insoluble substances floated and markedly prevented the partitioning operation. As a result of the HPLC analysis, the fulvene derivative and H-Phe-Ala-OtBu were not detected in the aqueous layer, and extraction and removal of the fulvene derivative could not be performed.

### Comparative Example 4

In the same manner as in Example 3, the operation was performed except that water (4 ml) and ethyl acetate (4 ml) were used as extraction and washing solvents instead of 90% aqueous acetonitrile-hexane. As a result of the HPLC analysis, the fulvene derivative was not detected in the aqueous layer, and the removal rate of the fulvene derivative into the aqueous layer was not more than 1%.

### Comparative Example 5

In the same manner as in Example 3, the operation was performed except that dimethylformamide (4 ml) and heptane (4 ml) were used as extraction and washing solvents instead of 90% aqueous acetonitrile-hexane. As a result of the HPLC analysis, H-Phe-Ala-OtBu was not detected in the heptane layer, and the removal rate of the fulvene derivative from the reaction mixture into the heptane layer was 44%.

### Comparative Example 6

In the same manner as in Example 3, the operation was performed except that 90% aqueous dimethylformamide (4 ml) and heptane (4 ml) were used as extraction and washing solvents instead of 90% aqueous acetonitrile-hexane. As a result of the HPLC analysis, H-Phe-Ala-OtBu was not detected in the heptane layer, and the removal rate of the fulvene derivative from the reaction mixture into the heptane layer was 51%. Industrial Applicability

The method of the present invention can be preferably utilized for industrial production of peptide pharmaceutical products and the like.

This application is based on patent application No. 193153/2007 filed in Japan, the contents of which are hereby incorporated in full herein by reference.
Although the present invention have been presented or described by referring to preferred embodiments of this invention, it will, however, be understood by those of ordinary skill in the art that various modifications may be made to the forms and details without departing from the scope of the invention as set forth in the appended claims. All patents, patent publications and other publications indicated or cited in the Specification are hereby incorporated in their entireties by reference.

## Claims

1. A method of removing dibenzofulvene and/or a dibenzofulvene amine adduct from a reaction mixture obtained by reacting an amino acid compound protected with an Fmoc group with an amine for deprotection, which comprises stirring and partitioning the reaction mixture in a hydrocarbon solvent having a carbon number of 5 or above and a polar organic solvent (excluding organic amide solvents) immiscible with the hydrocarbon solvent, and removing the hydrocarbon solvent layer in which the dibenzofulvene and/or the dibenzofulvene amine adduct are/is dissolved.

2. The method of claim 1, wherein the amine is selected from diethylamine, dimethylamine, piperidine, morpholine and 1,8-diazabicyclo[5.4.0]undecen-7-en (DBU).

3. The method of claim 2, wherein the amine is selected from diethylamine and dimethylamine.

4. The method of any of claims 1 to 3, wherein the hydrocarbon solvent is at least one kind selected from n-pentane, n-hexane, n-heptane, n-octane and decalin.

5. The method of any of claims 1 to 4, wherein the polar organic solvent comprises at least one kind selected from acetonitrile and methanol.

6. The method of any of claims 1 to 5, wherein the polar organic solvent further comprises water.

7. The method of any of claims 1 to 6, wherein the amino acid compound protected with the Fmoc group is an amino acid ester protected with an Fmoc group or a peptide protected with an Fmoc group.

8. A method of producing peptide by a liquid phase synthesis method, comprising the method of claim 7.

9. The method of claim 8, comprising
(1) a step of condensing C-protected peptide or C-protected amino acid with N-Fmoc amino acid in the presence of a condensing agent, and/or
(2) a step of condensing C-protected peptide or C-protected amino acid with N-Fmoc amino acid activated ester.

10. The method of claim 9, wherein the step (1) is performed in the further presence of an activator.

11. The method of claim 9 or 10, wherein an intermediate peptide obtained without isolation as a solid in claim 1 is subjected to the steps of claim 9.

12. The method of any of claims 8 to 11, which is performed by one-pot synthesis.
